# EUROPÄISCHE PATENTSCHRIFT

(11) **EP 1 214 042 B1**
(45) Veröffentlichungstag und Bekanntmachung des Hinweises auf die Patenterteilung: **21.07.2004**
(21) Anmeldenummer: 00958518.3
(22) Anmeldetag: 31.08.2000
(51) Int. Cl.: A61K 6/083

(54) **AUTOPOLYMERISIERENDE PROTHESEN- UND UNTERFÜTTERUNGSMATERIALIEN UND IHRE VERWENDUNG**
AUTOPOLYMERIZING PROSTHETICAL AND REBASE MATERIALS AND THEIR USE
MATERIAUX DE PROTHESES ET DE REBASAGE A AUTOPOLYMERISATION, ET LEUR UTILISATION

(30) Priorität: 02.09.1999 DE 19941829
(43) Veröffentlichungstag der Anmeldung: 19.06.2002
(73) Patentinhaber: VOCO GmbH, 27472 Cuxhaven (DE)
(72) Erfinder: PLAUMANN, Manfred, Thomas, D-27472 Cuxhaven (DE); EVERS, Christoph, D-27472 Cuxhaven (DE)
(74) Vertreter: Eisenführ, Speiser & Partner
(86) Internationale Anmeldenummer: PCT/EP2000/008489
(87) Internationale Veröffentlichungsnummer: WO 2001/017482

(56) Entgegenhaltungen:
- EP-A- 0 427 300
- EP-A- 0 442 326
- EP-A- 0 630 641
- DE-A- 19 635 667

## Beschreibung

Die Erfindung betrifft autopolymerisierende Prothesen- und/oder Unterfütterungsmaterialien.
Es ist bekannt, Dentalwerkstoffe auf Basis von polymeren Methacrylaten herzustellen. So werden beispielsweise Werkstoffe hergestellt, die Polymethylmethacrylat-Perlpolymerisate in einer Pulverkomponente und Mischungen aus Methylmethacrylat und Ethylmethacrylat in einer flüssigen Komponente enthalten. Die Mischungen härten im allgemeinen durch radikalische Polymerisation unter Formgebung aus (Ullmann'8 Enzyklopädia of Industrial Chemistry, 5^{th} Ed., Vol. A8, 1987). In einer Veröffentlichung von T. Arima et al. (Analysis of composition and structure of hard autopolymerizing reline resins; Journal of Oral Rehabilitation 23; 346 (1996)) werden beispielsweise verschiedene, autopolymerisierende, harte dentale Unterfütterungsmaterialien beschrieben. Alle beschriebenen Materialien sind Pulver/Flüssigkeits-Systeme, die als Hauptbestandteil in der Pulverkomponente Polymethylmethacrylate (PMMA) und/oder Polyethylmethacrylate (PEMA) und als Hauptbestandteil in der Flüssigkeit Monomethacrylat-Monomere (MMA, EMA etc.) enthalten.

Die als Pulver/Flüssigkeits-Systeme vorliegenden Polymerisate werden als chemoplastisch zu verarbeitende Materialien bezeichnet. Die chemoplastischen Materialien enthalten in der Flüssigkeit die zu polymerisierenden Monomerbestandteile und im Pulver anorganische und organische Füllstoffe. Die anorganischen Füllstoffe bestehen zumeist aus Stoffen wie Siliciumdioxid, Silikaten oder Gläser. Die organischen Füllstoffe sind in der Regel Polymere, die in unregelmäßiger Form oder in Kugelform vorliegen können. Kugelförmige Polymere werden als sogenannte Perlpolymerisate als Füllstoff in dentalen Werkstoffen aufgrund der Verbesserung der physikalischen Eigenschaften bevorzugt verwendet. Die Druckschriften EP-2850916, EP-442326, DE 2849280 und DE-2849936 beschreiben Perlpolymerisate und deren Verwendung in dentalen Werkstoffen. Beispielsweise bei der Aushärtung zu einem dentalen Prothesenkunststoff erfolgt eine Vemetzung der Polymerteilchen des Pulvers, z.B. dem Perlpolymerisat, mit in diese Teilchen während der Anquellphase eingedrungenen und später zu polymerisierenden Monomere der Flüssigkeitskomponente. Die Bereitstellung eines dentalen Werkstoffes als autopolymerisrerendes Paste-Paste-System ist aufgrund des Quellverhaltens der verwendeten Perlpolymerisate nicht möglich.

Der Anteil an diesen Monomeren in der Flüssigkeits-Komponente, zumeist sind es Methylmethacrylatmonomere, kann bis zu 90 Gew.% betragen. Monomethacrylat-Monomere stellen beim Polymerisationsprozeß und in den nicht vollständig ausgehärteten Werkstoffen ein hohes Allergiepotential dar.
In der EP-270915 werden polymerisierende Massen auf Basis von Methacrylatestern beschrieben, die als organischen Füllstoff ein Fällungspolymerisat in Form unregelmäßiger Agglomeratteilchen enthalten. Die bei einer Fällungspolymerisation entstehenden porösen Agglomerate bestehen aus kugeligen Primär- und unregelmäßigen Sekundärteilchen. Als Füllstoffe werden die Perlpolymerisate aufgrund ihres Quellverhaltens bzw. ihrer Absetzneigung als ungeeignet beschrieben.
In der EP-630640 sind dentale Zusammensetzungen zur Herstellung von Prothesenkunststoff beschrieben, wobei es sich um sogenannte heißhärtende, d.h. bei Zufuhr von Wärme polymerisierende, Einkomponenten-Materialien handelt. Die Materialien umfassen ebenfalls Polymere, die bei Kontakt mit den zu polymerisierenden Acrylatmonomeren aufquellen.
Bei den in EP 442326 zitierten Perlpolymerisaten handelt es sich um Perlpolymerisate, die durch anorganische Füllstoffe verstärkt sind. Durch das Inkorporieren mit anorganischen Füllstoff ergibt sich erst deren Einsatzmöglichkeit als Füllstoff in Dentalmaterialien. Es werden nur füllstoffhaltige Perlpolymerisate beschrieben und deren Anwendung in Zahnfüllmaterialien. Eine Verwendung von nicht quellenden Perfpolymerisaten in dentalen Prothesen- oder Unterfütterungsmaterialien wird nicht erwähnt.

Die in DE 19635667 beschriebenen Perlpolymerisate werden in dentalen lichthärtenden Opakern eingesetzt. Eine Verwendung in rein chemisch härtenden Prothesen- und Unterfütterungsmaterialien wird nicht offenbart.

In der EP-687451 werden dentale Prothesenkunststoffe und deren Herstellung beschrieben, wobei das Einkomponentenmaterial durch Bestrahlung mit Mikrowellen ausgehärtet wird.
Nachteil der Heißpolymerisate, zu denen auch die Mikrowellen-Polymerisate zählen, ist die erforderliche Bereitstellung der Öfen, Mikrowellengeräte und sonstigen Apparaturen sowie der hohe Zeitaufwand zur Aushärtung.

Neben den beschriebenen kalt- und heißhärtenden Polymerisaten als Pulver/Flüssigkeits-Systeme sind lichthärtende Systeme bekannt. Nachteil der lichthärtenden Systeme ist neben der Bereitstellung der Polymerisationslampe die Schrumpfungsneigung zur Strahlungsquelle hin. Dadurch können sich vor allem dickere Kunststoffobjekte vom Modell abziehen, oder es können sich, wenn der Kunststoff am Modell haftet, Spannungen aufbauen. Zudem ist bei lichthärtenden Systemen die Durchhärtetiefe begrenzt, was einen langwierigen schichtweisen Aufbau des Dentalkunststoffes erfordert.

Der nächstliegende Stand der Technik ist EP-427300, in der eine polymerisierbare Zusammensetzung beschrieben wird. Die wesentlichen Bestandteile dieser Zusammensetzung sind multifunktionelle, vernetzbare Vinylverbindungen, vernetzte Polymere in Form diskreter Partikel, die in Kontakt mit den polymerisierbaren Vinylverbindungen quellbar sind, und anorganische Füllstoffe.
Die beschriebene polymerisierbare Zusammensetzung kann u.a. als Prothesenmaterial oder Unterfütterungswerkstoff eingesetzt werden.
Nachteil der polymerisierbaren Zusammensetzung nach EP-427300 ist das Quellverhalten der Polymere, wenn sie in Kontakt mit den zu polymerisierenden Vinylverbindungen kommen. Die Bereitstellung eines lagerfähigen sofort gebrauchsfähigen Paste-Paste-Systems zur Herstellung von Prothesenkunststoff oder hartem Prothesenunterfütterungsmaterial ist nicht möglich.

Aufgabe der vorliegenden Erfindung ist es daher autopolymerisierendes Prothesen- und/oder Unterfütterungsmaterial zur Verfügung zu stellen, das
- die angeführten Nachteile dentaler Zusammensetzungen nicht aufweist,
- eine Alternative zu den vorhandenen dentalen Werkstoffen darstellt,
- insbesondere als Paste-Paste-System sofort gebrauchsfertig anzumischen ist,
- keinen zusätzlichen apparativen Aufwand zur Anmischung benötigt und
- kein bzw. ein äußerst minimiertes Allergiepotential aufweist.
Bei der Verwendung eines Prothesen- oder Unterfütterungsmaterials besteht ein zumeist großflächiger Kontakt mit den Schleimhäuten im Mund. Durch diesen großflächigen Kontakt gilt es Beeinträchtigungen durch das Material, z.B. Monomerfreisetzungen, zu minimieren.

Gelöst wird die Aufgabe durch ein autopolymerisierendes, rein chemisch-härtendes Prothesen- und/oder Unterfütterungsmaterial, das als wesentliche Bestandteile Perlpolymerisate und polymerisierbare Acrylatmonomere, ausgenommen Monomethacrylate, sowie übliche Zusatzstoffe enthält. Die Perlpolymerisate zeigen in Kontakt mit den Acrylatmonomeren keinerlei Quellverhalten und verändern dadurch die Viskosität der dentalen Zusammensetzung nicht.

Als Perlpolymerisate werden keine Fällungspolymerisate verwendet, da diese unregelmäßige Agglomeratteilchen bilden. Vorteilhafterweise werden hochvernetzte Suspensionsperlpolymerisate verwendet, wie beispielsweise PLEX 6690 F der Fa. Röhm GmbH, die im Kontakt mit den polymerisierbaren Acrylatmonomeren keinerlei Quellverhalten zeigen. Die Herstellung von Suspensionspolymerisaten ist aus dem Stand der Technik, wie beispielsweise aus B. Vollmert, Grundriss der makromolekularen Chemie, Band 1, 1988, Seite 180 ff, "Suspensionspolymerisation", bekannt. Vorteil der Suspensionspolymerisate gegenüber den Fällungspolymerisaten ist, daß keine porösen Agglomerate mit unregelmäßiger Partikelgröße entstehen.
Der Einsatz der Suspensionsperlpolymerisate in den erfindungsgemäßen Dentalmaterialien führt vorteilhafterweise zur Verbesserung der physikalischen Eigenschaften, insbesondere zu einer Erhöhung der Transparenz und zur Verbesserung der Polierbarkeit der Materialien. Aus diesem Grund werden auch keine füllstoffhaltigen Perlpolymerisate verwendet.

Als polymerisierbare Acrylatmonomere können bevorzugt folgende Methacrylate verwendet werden: Ethylenglykoldimethacrylat, Hexandioldimethacrylat, 1,3-Butandioldimethacrylat, Diethylenglykoldimethacrylat, Neopentylglykoldimethacrylat, 1,4-Butandioldimethacrylat, Triethylenglykoldimethacrylat, Trimethylolpropantrimethacrylat, Decandiol-1,10-dimethacrylat, Tetraethylenglykoldimethacrylat, Dodecandioldimethacrylat, Bisphenol-A-Glycidylmethacrylat (BisGMA), Ethoxy-BisGMA, Urethandimethacrylat, Polyethylenglykoi-200-dimethacrylat, Polyethylenglykol-400-dimethacrylat und/oder Polyethylenglykol-600-dimethacrylat.
Aufgrund des bevorzugten Einsatzes von Di- oder Trimethacrylaten und der Vermeidung von Monomethacrylaten wird die Restmonomeren-Konzentration und die Löslichkeit des Kunststoffmaterials verringert. Dadurch wird das Allergiepotential der dentalen Zusammensetzung und somit auch die Beeinträchtigungen oder Schädigungen des Anwenders oder des Patienten verhindert oder zumindest verringert.

Als übliche Zusatzstoffe können entsprechend dem Stand der Technik gegebenenfalls
- anorganische Füllstoffe, wie silanisierte und unsilanisierte Gläser, Glaskeramiken, Silikate, Siliciumdioxid, und Fluoro-Aluminium-Silicat-Gläser,
- Initiator- und Katalysatorsysteme, insbesondere Peroxyverbindungen, wie Benzoylperoxid, und Amin- oder Azoverbindungen,
- Stabilisatoren, Inhibitoren, Farbstoffe, Pigmente, Trübungsmittel und/oder Röntgenkontrastmittel verwendet werden.

Eine besonders bevorzugte Dentalzusammensetzung setzt sich im wesentlichen aus 10 - 30 Gew.% Perlpolymerisaten, 10 - 50 Gew.% polymerisierbaren Acrylatmonomeren, 0 - 5 Gew.% hochdispersem Siliciumdioxid, 10 - 50 Gew.% silanisierten Gläsern und 0,1 - 10 Gew.% Initiatoren zur chemischen Aushärtung zusammen.

Insbesondere ist die Verwendung der Prothesen- und/oder Unterfütterungsmaterialien als direkt anzumischendes Paste-Paste-System möglich, was bei den bekannten Zusammensetzungen nach dem Stand der Technik nicht möglich ist. Besonders bevorzugt ist die Verwendung der Dentalzusammensetzung als Paste-Paste-System in einem Verhältnis von 1 zu 1.
Es lassen sich somit Pasten für den Anwender in gebrauchsfertigen, sofort anzuwendenden Tuben-, Kartuschen- oder Schlauchbeutel-Systemen bereitstellen.
Geeignete Kartuschen- oder Schlauchbeutel-Systeme sind aus dem Stand der Technik zur Anmischung dentaler Materialien, wie beispielsweise Abformmassen, bekannt. Genannt seien zum Beispiel Anmischsysteme entsprechend den Druckschriften EP-630641, EP-669113, EP-261466, EP-294672, EP-664153 oder DE-29813684.

Zusammenfassend ergeben sich für die erfindungsgemäßen Prothesen- und/oder Unterfütterungsmaterialien folgende Vorteile:
- es ist ein rein chemisch-härtendes System,
- die eingesetzten Suspensionsperlpolymerisate zeigen keinerlei Quellverhalten,
- der Einsatz von nicht quellenden Perlpolymerisaten führt zur Verbesserung der Transluzenz und Polierbarkeit der Materialien,
- die Perlpolymerisate und zu polymerisierenden Monomere können innerhalb einer Paste bereitgestellt werden,
- es ist als Paste/Paste-System in Kartuschen anzuwenden, damit sofort gebrauchsfertig und Mischungsfehler, wie sie bei Pulver/Flüssigkeitssystemen auftauchen, werden vermieden
- es werden keine Monomethacrylate eingesetzt,
- Minimierung des Allergiepotentials,
- durch die Verwendung von Di- oder Trimethacrylate wird ein hoher Vernetzungsgrad bei der Polymerisation erzielt,
- es ist kein zusätzlicher apparativer Aufwand zur Aushärtung notwendig,
- eine standfeste Konsistenz der dentale Zusammensetzung nach dem Anmischen ist je nach Mischungsverhältnis problemlos einstellbar,
- die dentale Zusammensetzung weist gute physikalische Eigenschaften auf,
- bei der Verwendung als Prothesenkunststoff ist mit handelsüblichen Adhäsiven ein guter Verbund zu Kunststoffzähnen gegeben
- bei der Bereitstellung als Kartuschen- oder Schlauchbeutel-System ist eine direkte Applikation ohne zusätzlichen apparativen Aufwand und ein blasenfreies Anmischen möglich.

Die Erfindung soll anhand der nachfolgenden Beispiele näher erläutert werden.

### Beispiel 1:

Herstellung einer Dentalzusammensetzung zur Verwendung als hartes Prothesenunterfütterungsmaterial, indem 18,83 Gew.% Perlpolymerisat (PLEX 6690 F der Fa. Röhm GmbH), 31,2 Gew.% polymerisierbare Acrylatmonomere, bestehend aus einer Mischung aus 27 Gew.% Hexandioldimethacrylat, 25 Gew.% Urethandimethacrylat und 48 Gew.% BisGMA, 3,3 Gew.% hochdisperses Siliciumdioxid (HDK H 2000 der Fa. Wacker Chemie), 20,52 Gew.% silanisierte Glaskeramikfüllstoffe, 20,29 Gew.% Fluoro-Aluminium-Silicat-Gläser und 5,86 Gew.% eines Initiator-Systems bestehend aus 2,9 Gew.% Benzoylperoxid (BPO), 0,26 Gew.% Butylhydroxytoluol (BHT), 2,2 Gew.% N,N-Bis-ethyl-para-toluidin und 0,5 Gew.% Dimethylaminoethylmethacrylat, als Paste-Paste-System angemischt werden. Die Basis- und Katalysator-Pasten enthalten jeweils 50% des Harzes (Perlpolymerisat, polymerisierbare Acrylatmonomere) und der anorganischen Füllstoffe. Die Basis-Paste enthält zudem das BPO und 0,15 Gew.% des BHT, die Katalysator-Paste enthält die restlichen oben angeführten Initiatorverbindungen.

### Beispiel 2:

Herstellung einer Dentalzusammensetzung zur Verwendung als hartes Prothesenunterfütterungsmaterial entsprechend Beispiel 1. Zunächst werden Suspensionsperlpolymerisate entsprechend B. Vollmert, Grundriss der makromolekularen Chemie, Band 1, 1988, Seite 180 ff, "Suspensionspolymerisation", aus einer Harzmischung aus 27 Gew.% Hexandioldimethacrylat, 25 Gew.% Urethandimethacrylat und 48 Gew.% BisGMA hergestellt. 18,83 Gew.% der so hergestellten Perlpolymerisate werden, wie in Beispiel 1 beschrieben, anstelle des PLEX 6690 F der Fa. Röhm GmbH zur Herstellung der Dentalzusammensetzung eingesetzt.

Aus Beispiel 1 und 2 werden direkt anzumischenden und sofort gebrauchsfertigen Paste-Paste-Systemen hergestellt. Folgende Untersuchungen werden mit den Unterfütterungsmaterialien durchgeführt:
1. Scherhaftungsmessung auf Prothesenmaterial auf PMMA-Basis
2. Messung der Verarbeitungs- und Abbindezeit
3. Messung der Biegefestigkeit nach dem Aushärten entsprechend ISO 1567

1.
   Es werden 10 Probenkörperzylinder mit einem Durchmesser von 2,5 cm und einer Höhe von 1,5 cm aus einem Prothesenmaterial auf PMMA-Basis hergestellt. In einer vorgefertigten Form mit einem Durchmesser von 0,9 cm werden die Unterfütterungsmaterialien der Beispiele 1 und 2 auf das Prothesenmaterial nach Auftragen eines handelsüblichen Primers aufpolymerisiert. Es werden 10 Scherversuche nach einem Tag Wasserlagerung bei 37°C durchgeführt.
2.
   Die Verarbeitungs- und Abbindezeiten der nach obiger Vorschrift hergestellten harten Prothesenunterfütterungsmaterialien werden gemessen.
3.
   Entsprechend DIN-ISO 1567 wird die Biegefestigkeit der auspolymerisierter Materialien, die nach obiger Vorschrift hergestellt wurden, gemessen.

Die nachfolgende Tabelle gibt die ermittelten Versuchsparameter wieder.

**Tabelle 1:**

| | **Beispiel 1** | **Beispiel 2** |
|---|---|---|
| **Scherhaftung** | 14,9 MPa | 12,5 MPa |
| **Verarbeitungszeit** | 6'40 | 7'00 |
| **Abbindezeit** | 4'00 | 4'00 |
| **Biegefestigkeit** | 46,7 MPa | 65,0 MPa |

Die Beispiele zeigen, daß die erfindungsgemäße dentale Zusammensetzung außergewöhnlich gute physikalische Eigenschaften aufweist.
Das nach Beispiel 2 hergestellte Unterfütterungsmaterial zeigt zudem eine hohe Transparenz. Die erfindungsgemäße Zusammensetzung genügt damit auch den hohen ästhetischen Anforderungen, die an Prothesen- und/oder Unterfütterungsmaterialien gestellt werden.

## Patentansprüche

1. Autopolymerisierendes Prothesen- und/oder Unterfütterungsmaterial enthaltend Perlpolymerisate und polymerisierbare Acrylatmonomere, ausgenommen Monomethacrylate, sowie übliche Zusatzstoffe, **dadurch gekennzeichnet, daß** die Perlpolymerisate keine Fällungspolymerisate sind, keine anorganischen Füllstoffe enthalten und in Kontakt mit den Acrylatmonomeren kein Quellverhalten zeigen.

2. Autopolymerisierendes Prothesen- und/oder Unterfütterungsmaterial nach Anspruch 1, **dadurch gekennzeichnet, daß** die Perlpolymerisate reine Suspensionspolymerisate sind.

3. Autopolymerisierendes Prothesen- und/oder Unterfütterungsmaterial nach Anspruch 1, oder 2, **dadurch gekennzeichnet, daß** die polymerisierbaren Acrylatmonomere ausgewählt sind aus der Gruppe Ethylenglykoldimethacrylat, Hexandioldimethacrylat, 1,3-Butandioldimethacrylat, Diethytenglykoldimethacrylat, Neopentylglykoldimethacrylat, 1,4-Butandioldimethacrylat, Triethylenglykoldimethacrylat, Trimethylolpropantrimethacrylat, Decandiol-1,10-dimethacrylat, Tetraethylenglykoldimethacrylat, Dodecandioldimethacrylat, Bisphenol-A-Glycidylmethacrylat (BisGMA), Ethoxy-BisGMA, Urethandimethacrylat, Polyethylenglykol-200-dimethacrylat, Polyethylenglykol-400-dimethacrylat und/oder Polyethylenglykol-600-dimethacrylat.

4. Autopolymerisierendes Prothesen- und/oder Unterfütterungsmaterial nach Anspruch 1, 2 oder 3, **dadurch gekennzeichnet, daß** die üblichen Zusatzstoffe gegebenenfalls
- anorganische Füllstoffe, wie silanisierte und unsilanisierte Gläser, Glaskeramiken, Silikate, Siliciumdioxid, und Fluoro-Aluminium-Silicat-Gläser,
- Initiator- und Katalysatorsysteme, insbesondere Peroxyverbindungen, wie Benzoylperoxid, und Amin- oder Azoverbindungen,
- Stabilisatoren, Inhibitoren, Farbstoffe, Pigmente, Trübungsmittel und/oder Röntgenkontrastmittel umfassen.

5. Paste-Paste-System enthaltend autopolymerisierendes Prothesen- und/oder Unterfütterungsmaterial nach einem der Ansprüche 1 bis 4.

6. Paste-Paste-System nach Anspruch 5, **dadurch gekennzeichnet, daß** das Paste-Paste-System im Verhältnis 1 zu 1 vorliegt.

7. Paste-Paste-System nach Anspruch 5, **dadurch gekennzeichnet, daß** das Paste-Paste-System im Verhältnis 1 zu 5 vorliegt.

## Claims

1. Autopolymerizing prosthetic material and/or rebase material comprising bead polymers and polymerizable acrylate monomers, apart from monomethacrylates, and conventional additives, **characterized in that** the bead polymers are not precipitation polymers, comprise no inorganic fillers and exhibit, on contact with the acrylate monomers, no swelling behaviour.

2. Autopolymerizing prosthetic material and/or rebase material according to Claim 1, **characterized in that** the bead polymers are pure suspension polymers.

3. Autopolymerizing prosthetic material and/or rebase material according to Claim 1 or 2, **characterized in that** the polymerizable acrylate monomers are chosen from the group consisting of ethylene glycol dimethacrylate, hexanediol dimethacrylate, 1,3-butanediol dimethacrylate, diethylene glycol dimethacrylate, neopentyl glycol dimethacrylate, 1,4-butanediol dimethacrylate, triethylene glycol dimethacrylate, trimethylolpropane trimethacrylate, 1,10-decanediol dimethacrylate, tetraethylene glycol dimethacrylate, dodecanediol dimethacrylate, bisphenol A glycidyl methacrylate (BisGMA), ethoxy-BisGMA, urethane dimethacrylate, polyethylene glycol 200 dimethacrylate, polyethylene glycol 400 dimethacrylate and/or polyethylene glycol 600 dimethacrylate.

4. Autopolymerizing prosthetic material and/or rebase material according to Claim 1, 2 or 3, **characterized in that** the conventional additives optionally include:
- inorganic fillers, such as silanized and nonsilanized glass, glass ceramics, silicates, silicon dioxide and fluoro-alumino-silicate glass,
- initiator and catalyst systems, in particular peroxy compounds, such as benzoyl peroxide, and amine or azo compounds,
- stabilizers, inhibitors, dyes, pigments, opacifiers and/or X-ray contrast media.

5. Paste/paste system comprising autopolymerizable prosthetic material and/or rebase material according to one of Claims 1 to 4.

6. Paste/paste system according to Claim 5, **characterized in that** the paste/paste system is present in the ratio 1 to 1.

7. Paste/paste system according to Claim 5, **characterized in that** the paste/paste system is present in the ratio 1 to 5.

## Revendications

1. Matériaux de prothèse et de rebasage à autopolymérisation contenant des polymères en suspension et des monomères acrylate polymérisables, à l'exclusion des monométhacrylates, ainsi que les additifs usuels, **caractérisés en ce que** les polymères en suspension ne sont pas des polymères de précipitation, ne contiennent pas de charge inorganique et ne présentent aucun comportement de gonflement au contact des monomères acrylate.

2. Matériaux de prothèse et de rebasage à autopolymérisation selon la revendication 1, **caractérisés en ce que** les polymères en suspension sont des polymères en suspension purs.

3. Matériaux de prothèse et de rebasage à autopolymérisation selon la revendication 1 ou 2, **caractérisés en ce que** les monomères acrylate polymérisables sont choisis parmi le groupe du diméthacrylate d'éthylèneglycol, le diméthacrylate d'hexanediol, le diméthacrylate de 1,3-butanediol, le diméthacrylate de diéthylèneglycol, le diméthacrylate de néopentylglycol, le diméthacrylate de 1,4-butanediol, le diméthacrylate de triéthylèneglycol, le triméthacrylate de triméthylolpropane, le diméthacrylate de décanediol-1,10, le diméthacrylate de tétraéthylèneglycol, le diméthacrylate de dodécanediol, le méthacrylate de bisphénol-A-glycidyle (BisGMA), l'éthoxy-BisGMA, le diméthacrylate d'uréthanne, le diméthacrylate de polyéthylèneglycol-200, le diméthacrylate de polyéthylèneglycol 400 et/ou le diméthacrylate de polyéthylènegycol-600.

4. Matériaux de prothèse et de rebasage à autopolymérisation selon la revendication 1, 2 ou 3, **caractérisés en ce que** les additifs usuels comprennent le cas échéant :
- des charges inorganiques, comme du verre silanisé ou non silanisé, des céramiques de verre, un silicate, le dioxyde de silicium et du verre fluoroaluminium-silicate ;
- des systèmes d'initiateur et de catalyseur, en particulier des composés peroxy, comme le benzoylperoxyde et des composés aminés ou azo ;
- des stabilisants, des inhibiteurs, des colorants, des pigments, des opacifiants et/ou des agents de contraste pour les rayons X.

5. Systèmes pâte-pâte, contenant les matériaux de prothèse et de rebasage à autopolymérisation selon l'une des revendications 1 à 4.

6. Systèmes pâte-pâte selon la revendication 5, **caractérisés en ce que** le système pâte-pâte est présent en un rapport 1 à 1.

7. Systèmes pâte-pâte selon la revendication 5, **caractérisés en ce que** le système pâte-pâte est présent en un rapport 1 à 5.
